(19) Europäisches Patentamt
European Patent Office
Office européen des brevets

(11) Publication number: 0 115 216 B1

(12) EUROPEAN PATENT SPECIFICATION

(45) Date of publication of patent specification: 25.04.90

(51) Int. Cl.$^5$: A 61 K 33/10, A 23 L 1/30, C 01 F 11/18

(21) Application number: 83308002.1

(22) Date of filing: 29.12.83

(54) **Health promotion composition.**

(30) Priority: 28.12.82 JP 233889/82

(43) Date of publication of application: 08.08.84 Bulletin 84/32

(45) Publication of the grant of the patent: 25.04.90 Bulletin 90/17

(84) Designated Contracting States: CH DE FR GB IT LI NL SE

(56) References cited:
AU-B- 465 312
FR-A-2 129 972

PATENTS ABSTRACTS OF JAPAN, vol. 6, no. 223(C-133)(1101), 9th November 1982; & JP - A - 57 125 676 (HIDEKI SUGIOKA) 05-08-1982

PATENTS ABSTRACTS OF JAPAN, vol. 6, no. 244(C-138)(1122), 2nd December 1982; & JP - A - 57 144 949 (HIDEKI SUGIOKA) 07-09-1982

The file contains technical information submitted after the application was filed and not included in this specification

(73) Proprietor: Someya, Nobuo
16-6, Kami-Ikebukuro 3-chome Toshima-ku
Tokyo 171 (JP)

(72) Inventor: Someya, Nobuo
16-6, Kami-Ikebukuro 3-chome Toshima-ku
Tokyo 171 (JP)

(74) Representative: Moore, Anthony John et al
Gee & Co. Chancery House Chancery Lane
London WC2A 1QU (GB)

EP 0 115 216 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for producing a health promotion composition comprising coral sand as an effective ingredient and to a product obtained thereby.

Tap water is weakly acidic due to the presence therein of residual chlorine. In recent years the consumption of so-called acidic foodstuffs such as meat has increased in accordance with the generally improved diet of the population but such a diet is also attended by harmful effects: there is concern that tooth decay and tendency to bone fracture have increased due to calcium deficiency, particularly in babies and children, despite a remarkable improvement in their physique.

An aim of the present invention is to provide a composition which will promote health by remedying the above-mentioned dietary deficiencies.

I have discovered that coral sand obtained by grinding living skeletons and semi-fossils of hermatypic coral or reef-building coral (hereinafter referred to as "reef-building coral") contains calcium carbonate as a main component and a variety of other minerals required by the human body in ecologically-derived proportions. According to the present invention there is thus provided a process for producing a health promotion composition, comprising desalinating naturally-occurring coral sand, disinfecting and drying the desalinated coral sand by heating at a temperature of from 80 to 150°C, and then pulverising the disinfected and dried coral sand.

Coral sand, which is obtained from living skeletons and semi-fossils of reef-building coral, contains calcium carbonate ($CaCO_3$), as a main component (about 95%); magnesium, strontium, sodium, potassium, phosphorus and chlorine, which are important bio-elements; and trace quantities of essential inorganic vitamin elements such as iron, copper, zinc, manganese, cobalt, chromium and boron, as shown in Table 1 below.

TABLE 1

| Sample / Analyte | A | C | G | AA | BB |
|---|---|---|---|---|---|
| Boron (B) | ≦0.01 | do. | do. | do. | do. |
| Sodium (Na) | 0.26 | 0.25 | 0.33 | 0.29 | 0.32 |
| Magnesium (MgO) | 1.92 | 1.52 | 1.89 | 1.68 | 2.43 |
| Aluminum ($Al_2O_3$) | 0.036 | 0.088 | 0.026 | 0.030 | 0.028 |
| Silicon ($SiO_2$) | 0.067 | 0.32 | 0.044 | 0.078 | 0.071 |
| Phosphorus ($PO_4$) | 0.071 | 0.069 | 0.061 | 0.057 | 0.062 |
| Sulfur (S) | 0.19 | 0.17 | 0.17 | 0.20 | 0.19 |
| Chlorine (Cl) | 0.013 | 0.012 | 0.024 | 0.023 | 0.008 |
| Potassium (K) | 0.0084 | 0.0076 | 0.0094 | 0.0080 | 0.0092 |
| Calcium (CaO) | 53.33 | 53.48 | 53.14 | 53.16 | 53.09 |
| Titanium ($TiO_2$) | 0.0015 | 0.00096 | 0.00034 | 0.00041 | 0.00043 |
| Chromium (Cr) | 0.0022 | 0.0022 | 0.0021 | 0.0022 | 0.0020 |
| Manganese (Mn) | 0.0019 | 0.0019 | 0.00080 | 0.0011 | 0.00092 |

| Sample | A | C | G | AA | BB |
|---|---|---|---|---|---|
| Analyte | | | | | |
| Iron (Fe) | 0.0071 | 0.0087 | 0.0052 | 0.0061 | 0.0073 |
| Cobalt (Co) | 0.0012 | 0.0011 | 0.0016 | 0.0011 | 0.0012 |
| Nickel (Ni) | 0.0011 | 0.0013 | 0.0014 | 0.0013 | 0.0011 |
| Copper (Cu) | 0.00056 | 0.00056 | 0.00056 | 0.00050 | 0.00056 |
| Zinc (Zn) | 0.00034 | 0.00050 | 0.00046 | 0.00062 | 0.00046 |
| Strontium (Sr) | 0.32 | 0.38 | 0.44 | 0.45 | 0.85 |
| Molybdenum (Mo) | ≦0.00005 | do | do. | do. | do. |
| Carbonic acid ($CO_2$) | 40.8 | 40.9 | 40.5 | 40.7 | 40.5 |
| Ignition Loss (450°C/2h) | 2.85 | 2.57 | 3.05 | 3.01 | 2.56 |

The above results were obtained by Osaka Chemical Analysis Center Co., Ltd., on various coral sand samples, A, C, G being obtained from Ishigakijima Islands and AA and BB from Okinawa Main Island.

The elements shown in Table 1 above, which are contained in typical examples of naturally-occurring coral sand are accumulated and calcified through the activity of reef-building coral, i.e. coelenterates. Accordingly, coral sand has a chemical constitution of ecologically-derived proportions, unlike food additives such as calcium carbonate obtained purely by chemical treatment. Due to such ecological derivation, the composition of the present invention is safe for human consumption and is a good quality source of minerals, especially calcium.

The process for producing the composition in accordance with the present invention involves firstly washing naturally-occurring coral sand with water to desalinate it, then disinfecting and drying the desalinated coral sand at a temperature of from about 80 to about 150°C, preferably 90 to 120°C, and reducing the disinfected and dried coal sand to a particle size of 59 to 197 openings per linear cm (150 to 500 mesh), preferably 79 to 177 openings per linear cm (200 to 450 mesh). The pulverization can also be effected either by freeze drying the disinfected and dried coral sand at a temperature of from about −180°C to −200°C in a nitrogen atmosphere, or by grinding coral sand which has been kneaded together with sea water or fresh water.

The reef-building coral employed as a raw material for preparing coral sand is known to be the major factor in building coral reefs because of skeletogenesis or calcification promoted by the action of *Zooxanthella* or endozoic algae present in the body. The optimum water temperature for growth of the reef-building coral is between about 25° and about 29°C. Reef-building coral is geographically located generally in the tropical and sub-tropical zones. Representative examples of reef-building coral include coral belonging to the order Madreporaria, Helioporida of the order Coenothecalia, Tubipora of the order Stolonifera and Millepora of the order Milleporina.

The thus-obtained finely-divided coral sand powder is extremely finely porous and has high solubility in water. In consequence, it may be dissolved in water (the powder dissolves in the form of ions) as it is and the resultant solution can be provided as drinking water. Alternatively, the coral sand fine powder may be formulated for example, as granules, tablets, emulsions, pills or suspension concentrates in the presence or absence of binder. Granules are usually manufactured by agglomeration or impregnation techniques. Generally granules will contain 0.5 to 25% by weight of the coral sand fine powder and up to 10% by weight of additives, if necessary and desired, such as stabilizer, slow release modifier and binder. Tablets or pills may be manufactured in conventional manner by mixing with binder such as starch or gelatin and tabletting the mixture using a tabletting machine.

The composition produced by the process of the present invention may also contain other ingredients,

for example, various nutrients such as vitamins (vitamins A, B, C, E and F) and sugars (glucose, fructose, sucrose and maltose). Further, the composition may be employed as an additive to various foodstuffs.

The composition of the present invention is especially useful for furnishing calcium. In addition, the composition produced by the process of the present invention can also furnish, *inter alia*, magnesium, strontium, potassium, phosphorus and copper, which are bio-elements; and further trace quantities of essential inorganic vitamin elements such as iron, manganese and potassium. By dissolving the coral sand powder in, for example, tap water, a delicious mineral water exhibiting weakly alkaline properties and which is free from the so-called bleaching powder smell can be obtained. This is assumed to be because an acid ($H_3O^+$) released during the course of chlorination of tap water is neutralized by the action of the coral sand which comprises calcium carbonate as a main component, whereby the system is rendered weakly alkaline in the presence of calcium ions ($Ca^{2+}$), as shown below:

$$Cl_2 + H_2O \geq HCl + HClO \quad (1)$$

$$HCl + H_2O \geq H_3O^+ + Cl^- \quad (2)$$

$$HClO + H_2O \geq H_3O^+ + Cl^- + O\uparrow \quad (3)$$

$$2CaCO_3 + 2H_2O^+ \geq 2Ca^{2+} + 2HCO_3^- + 2H_2O \quad (4)$$

$$Cl_2 + H_2O + 2CaCO_3 \longrightarrow 2Ca^{2+} + 2HCO_3^- + 2Cl^- + O\uparrow \quad (5)$$

The composition produced by the process in accordance with the present invention is more effective when given to adults at a daily dose of 1.0 to 10 g.

From the foregoing it will be appreciated that the health promotion composition produced by the process of the present invention can improve diet which is inclined to contain a disproportionate amount of acidic foodstuffs. In particular, calcium which tends to be lacking in babies and children can be spontaneously replenished and at the same time the so-called inorganic vitamin elements are provided.

The present invention will be described in more detail with reference to the Examples below. Percentages are by weight, temperature is room temperature and pressure is atmospheric pressure, unless otherwise indicated.

Example 1

Coral sand A shown in Table 1, obtained from Ishigakijima Island, was washed with water to effect desalination. After the absence of chlorine had been confirmed by the Mohr test, the coral sand was then disinfected and dried by heating at about 100°C. The thus disinfected and dried coral sand was then ground at room temperature to pass a sieve of 79 to 138 openings per linear cm (200 to 350 mesh).

The resulting coral sand powder was dissolved in water to give a composition containing 20% of the coral sand. The composition exhibited a pH of about 7.1.

Example 2

Coral sand C shown in Table 1, obtained from Ishigakijima Island, was treated in a manner similar to Example 1 except that the grinding was performed by freeze drying the dried coral sand at a temperature of from −180° to −200°C in a nitrogen flow. The resulting coral sand powder had a particle size of 79 to 118 openings per linear cm (200 to 300 mesh). An aqueous solution of the coral sand powder exhibited a pH of about 7.2.

**Claims**

1. A process for producing a health promotion composition, comprising desalinating naturally-occurring coral sand, disinfecting and drying the desalinated coral sand by heating at a temperature of from 80 to 150°C, and then pulverising the disinfected and dried coral sand.

2. A process as claimed in Claim 1, wherein the disinfected and dried coral sand is pulverised to a fine powder of a particle size passing 59 to 197 openings per linear cm (150 to 500 mesh).

3. A process as claimed in Claim 2, wherein the disinfected and dried coral sand is pulverised to a particle size passing 79 to 177 openings per linear cm (200 to 450 mesh).

4. A process as claimed in Claim 1, 2 or 3, wherein the pulverization is performed by grinding at room temperature.

5. A process as claimed in Claim 1, 2 or 3, wherein the pulverization is performed by freeze drying the disinfected and dried coral sand at a temperature of from −180 to −200°C in a nitrogen atmosphere.

6. A health promotion composition when prepared by a process as claimed in any of the preceding claims.

7. A composition as claimed in Claim 6, wherein said coral sand is present together with a binder and the composition is formulated as tablets.

8. A composition as claimed in Claim 7, wherein said binder is selected from starch and gelatin.

9. Use of the composition as claimed in Claim 6, 7 or 8 as an additive for food or drink or as a dietetic supplement.

**Patentansprüche**

1. Verfahren zur Herstellung eines gesundheitsfördernden Mittels bestehend aus den Schritten des Entsalzens von in der Natur vorkommenden Korrallensandes, dem Desinfizieren und Trocknen des entsalzenen Korallensandes durch Erhitzen auf eine zwischen 80 und 150°C liegende Temperatur und dann des anschliessenden Pulverisierens des desinfizierten und getrockneten Korallensandes.

2. Verfahren nach Patentanspruch 1, in dem der desinfizierte und getrocknete Korallensand auf ein feines Pulver mit einer solchen Grösse der Teilchen pulverisiert wird, dass diese durch ein Sieb mit 59 bis 197 Oeffnungen pro Längenzentimeter (150 bis 500 Maschen) hindurchgehen.

3. Verfahren nach Patentanspruch 2, in dem der desinfizierte und getrocknete Korallensand auf eine Grösse der Teilchen pulverisiert wird, dass diese durch ein Sieb mit 79 bis 177 Oeffnungen pro Längenzentimeter (200 bis 250 Maschen) hindurchgehen.

4. Verfahren nach Patentanspruch 1, 2 oder 3, in dem die Pulverisierung durch Schleifen bei Raumtemperatur erfolgt.

5. Verfahren nach Patentanspruch 1, 2 oder 3, in dem die Pulverisierung durch Gefriertrocknen des desinfizierten und getrockneten Korallensandes bei einer Temperatur von −180 bis −200°C in einer Stickstoffatmosphäre erfolgt.

6. Das gemäss einem der vorhergehenden Patentansprüche hergestellte, gesundheitsfördernde Mittel.

7. Mittel nach Patentanspruch 6, wobei der besagte Korallensand zusammen mit einem Bindemittel vorhanden ist und daraus Tabletten hergestellt werden.

8. Mittel nach Patentanspruch 7, in der das besagte Bindemittel aus Stärke und Gelatine ausgewählt wird.

9. Verwendung des Mittels nach Patentanspruch 6, 7 oder 8 als ein Zusatz in Nahrungsmitteln oder Getränken oder als Diätzusatz.

**Revendications**

1. Procédé de préparation d'une composition pour améliorer la santé comprenant de dessaler du sable de corail naturel, de désinfecter et de sécher le sable de corail dessalé en le chauffant à une température comprise entre 80 et 150°C et ensuite de réduire en poudre le sable de corail désinfecté et séché.

2. Procédé selon la revendication 1, où le sable de corail désinfecté et séché est réduit en une poudre fine ayant une granulométrie lui permettant de traverser un tamis ayant de 59 à 197 ouvertures par cm linéaire (150 à 500 mesh).

3. Procédé selon la revendication 2, où le sable de corail désinfecté et séché est réduit en une poudre ayant une granulométrie lui permettant de traverser un tamis ayant de 79 à 177 ouvertures par cm linéaire (200 à 450 mesh).

4. Procédé selon la revendication 1, 2 ou 3, où la réduction en poudre est réalisée par broyage à la température ambiante.

5. Procédé selon la revendication 1, 2 ou 3, où la réduction en poudre est réalisée par lyophilisation du sable de corail désinfecté et séché à une température comprise entre −180 et −200°C dans une atmosphère d'azote.

6. Composition pour améliorer la santé préparée par un procédé selon l'une quelconque des revendications précédentes.

7. Composition selon la revendication 6, où ledit sable de corail est associé à un liant et où la composition est mise en comprimés.

8. Composition selon la revendication 7, où ledit liant est choisi parmi l'amidon et la gélatine.

9. Utilisation de la composition selon la revendication 6, 7 ou 8 en tant qu'additif pour des aliments ou des boissons, ou en tant que supplément diététique.